# EUROPEAN PATENT SPECIFICATION

(11) **EP 0 730 879 B1**
(45) Date of publication and mention of the grant of the patent: **30.10.2002**
(21) Application number: 96200357.0
(22) Date of filing: 14.02.1996
(51) Int. Cl.: A61M 25/00

(54) **Balloon catheter and method for manufacturing such a catheter**
Ballonkatheter und Verfahren zur Herstellung eines solchen Katheters
Cathéter à ballon et procédé de fabrication d'un tel cathéter

(30) Priority: 08.03.1995 NL 9500468
(43) Date of publication of application: 11.09.1996
(73) Proprietor: CORDIS EUROPA N.V., NL-9301 LJ Roden (NL)
(72) Inventor: Hylkema, Lucas Johannes, 9716 CW Groningen (NL)
(74) Representative: 't Jong, Bastiaan Jacob

(56) References cited:
- EP-A- 0 304 258
- EP-A- 0 376 451
- EP-A- 0 439 202
- US-A- 4 906 244
- US-A- 4 935 190
- US-A- 5 015 230

## Description

The invention relates to a method for manufacturing a balloon catheter, in particular a balloon catheter with a very large balloon, as is known from US patent application 4 906 244.

The balloon for such a balloon catheter is made up of a central section with on both sides transition sections to tube-like end sections. The balloon is usually manufactured by means of blow-moulding a piece of tube-like semimanufacture. The wall of the semimanufacture is relatively thick, so that sufficient material is available for the central section which is to be expanded.

In the transition sections the wall thickness decreases from that of the tube-like end section to that of the expanded central section. The wall of the transition section close to the end section is consequently still relatively thick, which considerably impedes the folding of the balloon into a small diameter. This folding into a small diameter is desirable however, in order to be able to introduce the balloon catheter properly into a patient.

What is more, the wall thickness in the transition section does, in general, not decrease uniformly. There will be sections with a relatively thick wall separated from each other by sections with a relatively thin wall. This further impedes the folding of the balloon into a small diameter.

The object of the invention is to provide a method for manufacturing such a balloon catheter, resulting in a balloon which can be folded into a small diameter properly.

This aim is achieved with the method as specified in claim 1. By receiving the semi-manufactured product thus twisted in the mould, ridges of material extending fan-shaped from the end section are formed on expansion in the transition section of the balloon member, of which the walls are thicker than those of the intermediate sections. As a result the transition section can be folded together very easily into a small diameter.

The tube-like semimanufacture is preferably manufactured by extrusion and is pre-stretched before it is received in the mould. As a result the material will obtain the optimum properties required for the ensuing blow-moulding process.

An advantageous further development is specified in claim 3. By carrying out the blow-moulding process in two stages, an optimum distribution of material can be achieved in the end product.

With the measures as set out in claim 4, a suitable number of evenly distributed fan-shaped ridges of material is obtained. There are approximately ten of them.

A further advantageous development is specified in claim 5. As a result the ridges of material stretch out fan-shaped in a pattern of spirals extending in a clockwise direction. The introduction of the catheter into the patient and the removal thereof later on, can be facilitated by rotating the catheter round its longitudinal axis. The ridges of material extending in the folded state in a helical pattern support, on rotation, the movement in a longitudinal direction by a screw action.

The invention also relates to and provides a balloon catheter manufactured in accordance with the method as specified in claim 6.

An advantageous small diameter of the balloon member can be achieved with the measures as set out in claim 7. A very gradual transition without bulges is achieved from the end sections to the folded central section.

Additional advantageous properties and advantages of the invention will become apparent when reading the following description of an example of an embodiment with reference to the attached drawings.
- Figure 1: shows a catheter manufactured with the method according to the invention in a partly broken away perspective view,
- Figure 2: illustrates schematically one step of the method according to the invention,
- Figure 3: is a large-scale drawing of the balloon member of the catheter of figure 1,
- Figure 4: shows a frontal view of the balloon member of figure 3 in the direction as indicated by arrow IV,
- Figure 5: shows the balloon member of figure 3 in the folded state,
- Figure 6: shows a cross-section along the line VI-VI of figure 5.
- Figure 7: shows a catheter with an introduction sheath

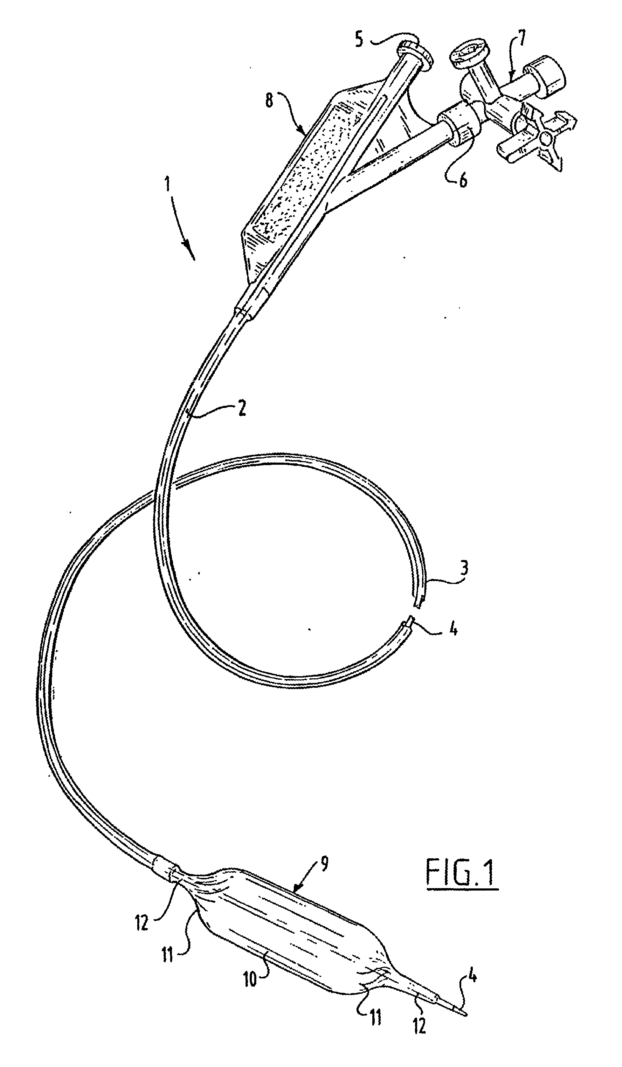

The catheter 1 shown in figure 1 comprises a tube-like basic body 2 which has been assembled from an outer tube-like element 3, in a central lumen whereof an inner tube-like element 4 has been received. The tube-like element 4 also comprises a lumen.

At the proximal end of the catheter 1 a connecting element 8 has been arranged. This connecting element 8 has two connections 5 and 6. The connection 5 is connected with the lumen of the inner tube-like element 4 and the connection 6 is connected with the lumen of the outer tube-like element 3, that is to say, the channel with annular cross-section formed by the space not taken up by the inner tube-like element 4 inside the lumen of the outer tube-like element 3.

As can be seen in the figure, a tap 7, known as such, can be arranged to the connection 6.

At the distal end the catheter 1 is provided with a balloon member 9. This is a balloon with a very large diameter sometimes referred to as a "fatsy".

The balloon member 9 has an active central section 10 with on either side transition sections 11 turning into end sections 12.

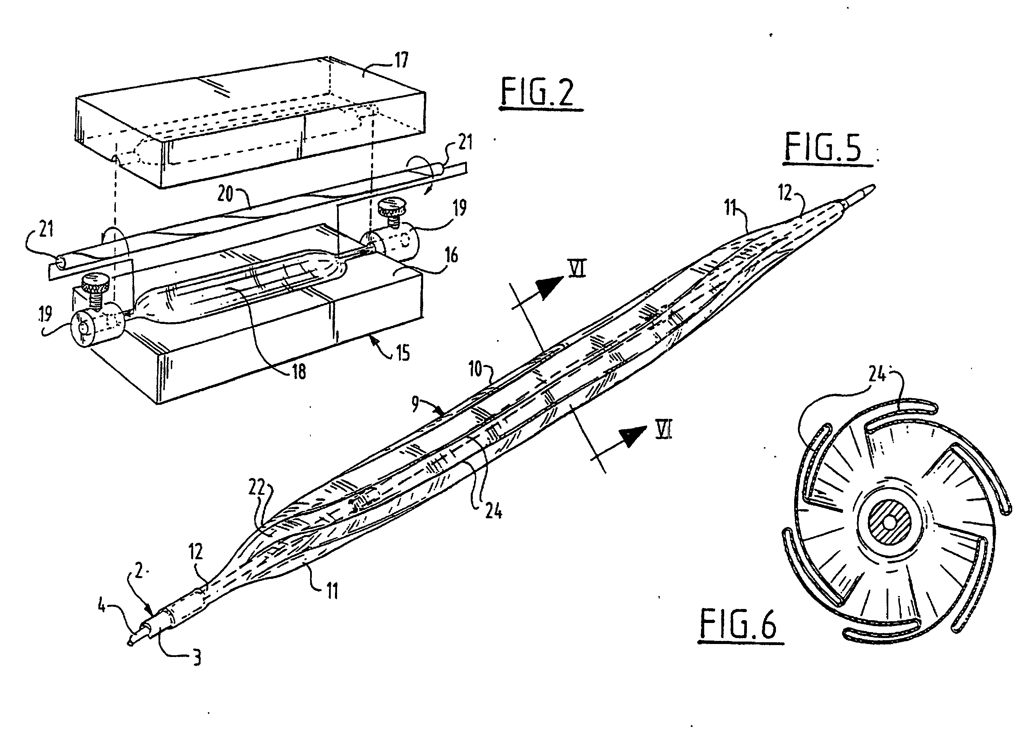

The balloon member 9 has been manufactured in at least one step of the blow-moulding process by the method according to the invention. This step will be explained in greater detail with reference to figure 2.

In figure 2 a mould 15 has been illustrated schematically, comprising two mould sections 16, 17. In the mould sections 16 and 17 a mould cavity 18 has been provided, the shape of which corresponds with that of an intended expanded form of the balloon member to be manufactured. At opposite ends this mould cavity 18 turns into securing elements 19. In the securing elements 19 the end sections 21 of a tube-like semimanufacture 20 are secured.

As is indicated by the arrows, the semimanufacture 20 is twisted before it is received in the mould 15. For this purpose the end sections 21 are turned over a certain angle in relation to each other. A suitable angle is one of 270°.

After receiving the semimanufacture 20 in the mould 15, a pressure difference between the inside and the outside of the semimanufacture 20 is generated in an obvious manner not explained in greater detail here, for instance by connecting the channel defined inside the semimanufacture 20 to a source of gas under pressure. At the same time the semimanufacture is heated to a temperature higher than its softening temperature, so that it will be "blown up". The inflated section of the semimanufacture 20 arranges itself against the wall of the mould cavity 18 and thus obtains the intended expanded form.

Next one can allow the semimanufacture to cool down, so that the expanded form is retained. Because of the flexibility of the material of which the semimanufacture has been made, which is a plastic material, the balloon member formed can be folded and expanded again afterwards by increasing the pressure inside.

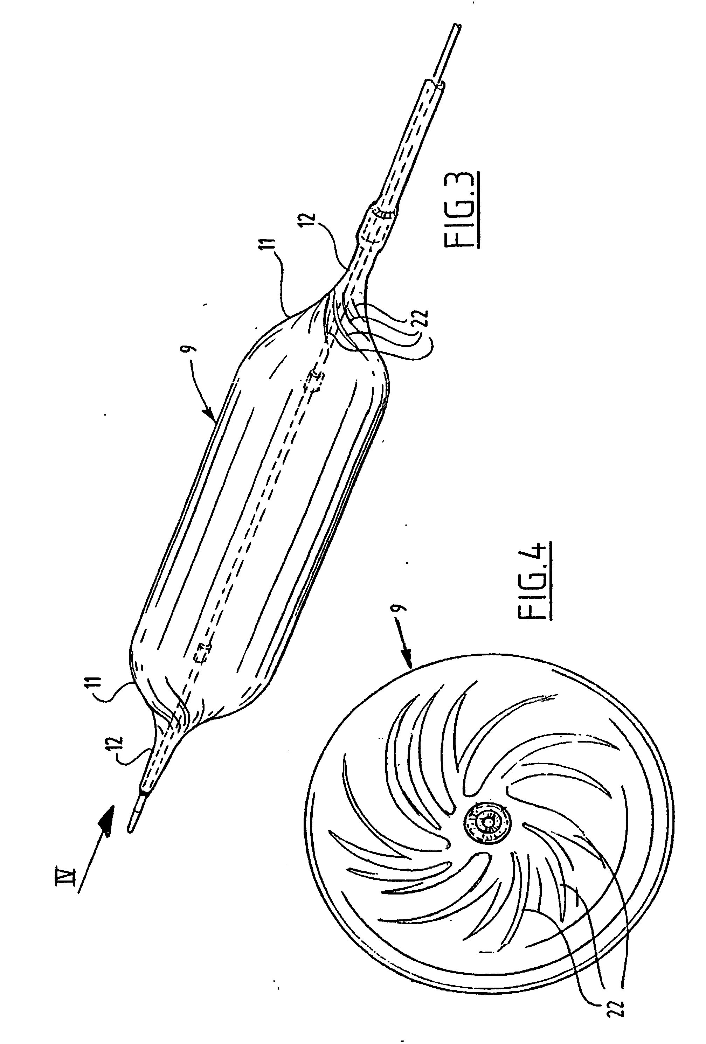

Figure 3 is a large-scale drawing of the balloon member thus formed. Because the semimanufacture 20 has been received in the mould 15 in the twisted manner described above, ridges of material 22 have been formed in the transition sections 11 extending fan-shaped from the end sections 12. The ridges of material 22 are relatively thick, whereas the material in between is stretched out. The ridges of material 22 have been shown once more in figure 4 for the sake of clarity.

The ridges of material 22 can, to a certain extent, be compared with the spokes of an umbrella. They can fold against each other, whereby the thinner material in-between is folded into pleads. Thus, in folded state, a small diameter can be achieved.

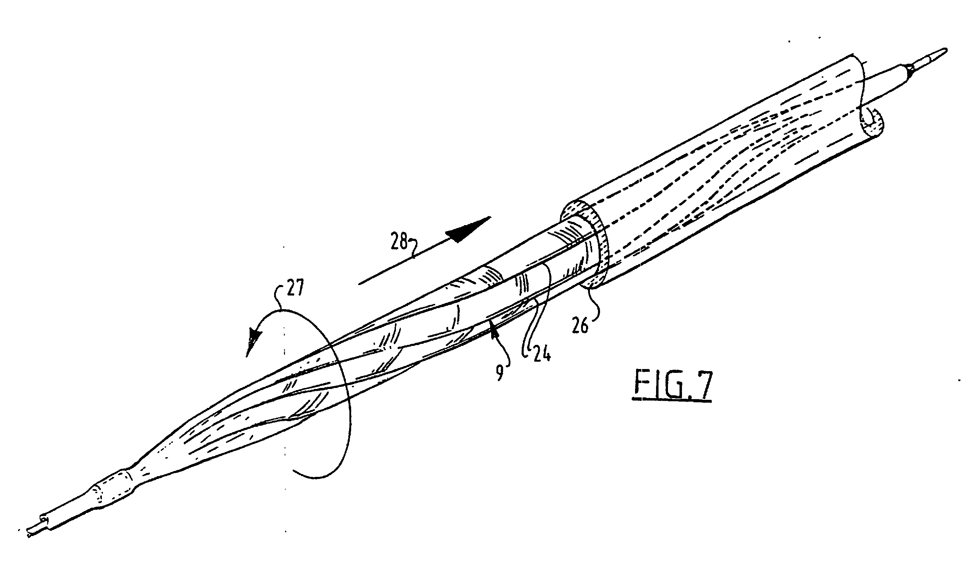

This folded state is illustrated in the figures 5 up to and including 7.

As can be seen in the figures 5 and 6, the central section 10 and the transition sections 11 are folded in pleads against the inner tube-like element 4 of the basic body 2. The folds 24 fit closely together and substantially coincide with the fan-shaped ridges of material 22.

As can be seen in figure 5 as well, the outer tube-like element 3 of the basic body 2 is shorter than the inner tube-like element 4. The relatively proximal end section 12 of the balloon member 9 is connected with the end of the outer tube-like element 3, whereas the relatively distal end section 12 of the balloon member 9 is connected with the inner tube-like element 4. The inside of the balloon member 9 is therefore connected via the remaining channel with annular cross-section inside the outer tube-like element 3 with the connection 5 of the connecting member 8. By supplying via this connection a gas or liquid under pressure, the balloon 9 can be unfolded into its expanded form. This occurs following introduction of the catheter into a patient for the purpose of dilation or occlusion of a blood vessel.

Introduction occurs in the usual manner via an introduction sheath 26 which has been illustrated schematically in figure 7. This introduction sheath 26 can have a relatively small inside diameter, as the balloon member 9 can be folded into a small diameter and because no bulges are formed at the transition sections as a result of unevenly distributed basic material.

As a result of the ridges of material extending in a fan-shaped manner and the closely fitting folds, sections with a helically shaped profile are formed on either side of the balloon member. By rotating the catheter in a suitable manner, as indicated by arrow 27, a certain force 28 can be generated by screw action, which facilitates the introduction of the catheter. Also the removal of the catheter can take place smoothly by applying a correct rotation.

Although the method according to the invention is explained with reference to figure 2, in which a balloon member is formed in one single blow-moulding step, it is also possible to achieve the same in more steps. A first initiation can for example be carried out in the form of a limited expansion in order to obtain a second semimanufacture with a suitable material distribution. This second semimanufacture can then be expanded in a second blow-moulding step to obtain the intended ultimate form. In that case the suitable material distribution is achieved during the first step by the right choice of mould cavity of the mould in which this step is performed.

## Claims

1. Method for manufacturing a balloon catheter, comprising the providing of a piece of tube-like basic material, the manufacturing of a balloon member and the connecting of the balloon member with the basic material, wherein the manufacturing of the balloon member comprises the providing of a mould (15) which in its turn has been provided with a mould cavity (18) corresponding to an intended expanded form of the balloon member, turning at opposite ends into securing elements (19) for the purpose of securing opposite end sections (21) of a piece of tube-like semimanufacture (20) inside the mould cavity (18), the receiving of the semimanufacture (20) in the mould (15), the heating of the semimanufacture (20), the generating of a pressure difference between the inside and the outside of the semimanufacture (20) as a result of which it expands into the balloon member arranged against the walls of the mould cavity (18), **characterised in that** the end sections (21) of the semimanufacture (20) on being received in the mould (15) are turned over an angle in relation to each other.

2. Method as claimed in claim 1, wherein the tube-like semimanufacture (20) is manufactured by means of extrusion and is pre-stretched before being received in the mould (15).

3. Method as claimed in one of the previous claims, wherein the intended expanded form is an intermediate form, and the heated, expanded balloon member is transferred to a second mould in which it is given its definite shape, and the cooling down of the balloon member.

4. Method as claimed in one of the previous claims, wherein the end sections (21) are turned substantially over an angle of 270° in relation to each other.

5. Method as claimed in one of the previous claims, wherein the end sections (21) are turned in such a direction, that the semimanufacture is twisted in the direction of a helix extending clockwise.

6. Balloon catheter manufactured with the method according to one of the previous claims, wherein the balloon member comprises a central section (10) with on either side transition sections (11) turning into tube-like end sections (12), **characterised in that** at least one of the transition sections (11) comprises ridges (22) of material extending fan-shaped from the end section (12).

7. Balloon catheter as claimed in claim 6, wherein the central section (10) and the transition sections (11) are folded in pleads against the basic body and wherein the pleads substantially coincide with the fan-shaped ridges (22) of material.

8. Balloon catheter as claimed in claim 6 or 7, wherein the ridges (22) of material are substantially evenly distributed around the circumference of the transition section (11).

9. Balloon catheter according to one of the claims 6-8, wherein the transition section (11) comprises between 5 and 15 ridges (22) of material.

10. Balloon catheter as claimed in claim 9, wherein the transition section (11) comprises some 10 ridges (22) of material.

## Patentansprüche

1. Verfahren zur Herstellung eines Ballonkatheters, aufweisend das Vorsehen eines Teils aus röhrartigem Basismaterial, das Herstellen eines Ballonteils und das Verbinden des Ballonteils mit dem Basismaterial, wobei das Herstellen des Ballonteils das Vorsehen einer Form (15), welche ihrerseits mit einer Formkammer (18) versehen wurde, die einer beabsichtigten expandierten Form des Ballonteils entspricht und an einander gegenüberliegenden Enden in Befestigungselemente (19) zum Zwecke der Befestigung einander gegenüberliegender Endabschnitte (21) eines Teils eines röhrenartigen Halbfertigfabrikats (20) an der Innenseite der Formkammer (18) übergeht, das Aufnehmen des Halbfertigfabrikates (20) in die Form (15), das Erhitzen des Halbfertigfabrikates (20), und das Erzeugen einer Druckdifferenz zwischen der Innenseite und der Außenseite des Halbfertigfabrikates (20) umfaßt, infolgedessen es zu dem Ballonteil expandiert, das an die Wände der Formkammer (18) angelegt wird, **dadurch gekennzeichnet, dass** die Endabschnitte (21) des Halbfertigfabrikates (20), das in der Form (15) aufgenommen wird, in einem Winkel relativ zueinander verdreht werden.

2. Verfahren nach Anspruch 1, wobei das röhrenartige Halbfertigfabrikat (20) mittels Extrudieren hergestellt wird und vorgedehnt wird, bevor es in der Form (15) aufgenommen wird.

3. Verfahren nach einem der vorhergehenden Ansprüche, wobei die beabsichtigte expandierte Form eine Zwischenform ist und das erhitzte expandierte Ballonteil in eine zweite Form übergeben wird, in welcher ihm seine endgültige Form gegeben wird, und das Abkühlen des Ballonteils.

4. Verfahren nach einem der vorhergehenden Ansprüche, wobei die Endabschnitte (21) im wesentlichen im Winkel von 270° relativ zueinander verdreht werden.

5. Verfahren nach einem der vorhergehenden Ansprüche, wobei die Endabschnitte (21) in einer solchen Richtung verdreht werden, dass das Halbfertigfabrikat in der Richtung einer sich im Uhrzeigersinn erstreckenden Spirale verdreht wird.

6. Ballonkatheter, der nach dem Verfahren gemäß einem der vorhergehenden Ansprüche hergestellt wird, wobei das Ballonteil einen Mittelabschnitt (10) aufweist, wobei an jeder Seite Übergangsabschnitte (11) zu röhrenartigen Endabschnitten (12) übergehen, **dadurch gekennzeichnet, dass** mindestens einer der Übergangsabschnitte (11) Materialwülste (22) aufweist, die sich fächerförmig von dem Endabschnitt (12) erstrecken.

7. Ballonkatheter nach Anspruch 6, wobei der Mittelabschnitt (10) und die Übergangsabschnitte (11) in Falten gegen den Basiskörper gefaltet sind, und wobei sich die Falten im wesentlichen mit den fächerförmigen Materialwülsten (22) decken.

8. Ballonkatheter nach Anspruch 6 oder 7, wobei die Materialwülste (22) im wesentlichen gleichmäßig um den Umfang des Übergangsabschnitts (11) herum verteilt sind.

9. Ballonkatheter nach einem der Ansprüche 6-8, wobei der Übergangsabschnitt (11) zwischen 5 und 15 Materialwülste (22) aufweist.

10. Ballonkatheter nach Anspruch 9, wobei der Übergangsabschnitt (11) etwa 10 Materialwülste (22) aufweist.

## Revendications

1. Procédé de fabrication d'un cathéter à ballon, comprenant l'approvisionnement d'un morceau de matière de base de forme tubulaire, la fabrication d'un élément formant ballon et l'assemblage de l'élément formant ballon à la matière de base, dans lequel la fabrication de l'élément formant ballon comprend la présence d'un moule (15) qui, quant à lui, a été pourvu d'une cavité de moulage (18) correspondant à une forme dilatée prévue pour l'élément formant ballon, la rotation aux extrémités opposées dans des éléments de fixation (19) afin de fixer des parties d'extrémité opposées (21) d'un morceau de semi-produit tubulaire (20) à l'intérieur de la cavité de moulage (18), la mise en place du semi-produit (20) dans le moule (15), le chauffage du semi-produit (20), la création d'une différence de pression entre l'intérieur et l'extérieur du semi-produit (20), pour ainsi aboutir à sa dilatation dans l'élément formant ballon, disposé contre les parois de la cavité de moulage (18), **caractérisé en ce que** les parties d'extrémité (21) du semi-produit (20) sont tournées, lors de leur réception dans le moule (15), suivant un certain angle l'une par rapport à l'autre.

2. Procédé selon la revendication 1, dans lequel le semi-produit tubulaire (20) est fabriqué par extrusion et est pré-étiré avant d'être mis en place dans le moule (15).

3. Procédé selon l'une des revendications précédentes, dans lequel la forme dilatée prévue est une forme intermédiaire, et l'élément formant ballon est transféré, à l'état chauffé et dilaté, vers un deuxième moule dans lequel lui est conférée sa forme définitive, avec ensuite refroidissement de l'élément formant ballon.

4. Procédé selon l'une des revendications précédentes, dans lequel les parties d'extrémité (21) sont tournées sur un angle de sensiblement 270° l'une par rapport à l'autre.

5. Procédé selon l'une des revendications précédentes, dans lequel les parties d'extrémité (21) sont tournées dans un sens tel que le produit semi-fini soit torsadé dans le sens d'une hélice s'étendant dans le sens des aiguilles d'une montre.

6. Cathéter à ballon, fabriqué par le procédé selon l'une des revendications précédentes, dans lequel l'élément formant ballon comprend une partie centrale (10) avec, de part et d'autre, des parties de transition (11) qui se transforment en des parties tubulaires d'extrémité (12), **caractérisé en ce qu'**au moins l'une des parties de transition (11) comporte des crêtes (22) de matière, qui s'étendent en forme d'éventail à partir de la partie d'extrémité (12).

7. Cathéter à ballon selon la revendication 6, dans lequel la partie centrale (10) et les parties de transition (11) sont pliées afin de constituer des plis contre le corps de base, et dans lequel les plis coïncident sensiblement avec les crêtes formant éventail (22) de la matière.

8. Cathéter à ballon selon la revendication 6 ou 7, dans lequel les crêtes (22) de la matière sont réparties sensiblement régulièrement sur toute la circonférence de la partie de transition (11).

9. Cathéter à ballon selon l'une des revendications 6 à 8, dans lequel la partie de transition (11) comprend entre 5 et 15 crêtes (22) de la matière.

10. Cathéter à ballon selon la revendication 9, dans lequel la partie de transition (11) comprend environ 10 crêtes (22) de la matière.
